(19) Europäisches Patentamt
European Patent Office
Office européen des brevets

(11) **EP 1 903 991 B1**

(12) **EUROPEAN PATENT SPECIFICATION**

(45) Date of publication and mention
of the grant of the patent:
**09.09.2009 Bulletin 2009/37**

(21) Application number: **06787430.5**

(22) Date of filing: **14.07.2006**

(51) Int Cl.:
*A61F 2/24* (2006.01)     *A61B 17/00* (2006.01)

(86) International application number:
**PCT/US2006/027519**

(87) International publication number:
**WO 2007/011799 (25.01.2007 Gazette 2007/04)**

(54) **Apparatus for remodelling a cardiac valve annulus**

Vorrichtung zur Ummodellierung eines Herzklappenrings

Appareil de remodelage de l'anneau d'une valve cardiaque

(84) Designated Contracting States:
**AT BE BG CH CY CZ DE DK EE ES FI FR GB GR HU IE IS IT LI LT LU LV MC NL PL PT RO SE SI SK TR**

(30) Priority: **15.07.2005 US 699730 P**

(43) Date of publication of application:
**02.04.2008 Bulletin 2008/14**

(73) Proprietor: **THE CLEVELAND CLINIC FOUNDATION**
**Cleveland, OH 44195 (US)**

(72) Inventors:
• **NAVIA, Jose, L.**
**Shaker Heights, OH 44122 (US)**

• **NAVIA, Jose, A.**
**Buenos Aires 1011 (AR)**

(74) Representative: **Naismith, Robert Stewart et al**
**Marks & Clerk LLP**
**Aurora**
**120 Bothwell Street**
**Glasgow**
**G2 7JS (GB)**

(56) References cited:
**WO-A-02/03892       WO-A2-02/15798**
**WO-A2-02/28321**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

The present invention relates generally to cardiovascular valve repair, and more particularly to an apparatus and method for remodeling a cardiac valve annulus.

### Background of the Invention

Mitral and tricuspid valve replacement and repair are traditionally performed with suture techniques. During valve replacement, sutures are spaced around the annulus (the point where the valve leaflet attaches to the heart) and then the sutures are attached to a prosthetic valve. The valve is lowered into position and when the sutures are tied, the valve is fastened to the annulus. The surgeon may remove all or part of the valve leaflets before inserting the prosthetic value. In valve repair, a diseased valve is left *in situ* and surgical procedures are performed to restore its function.

Frequently, an annuloplasty ring is used to reduce the size of the annulus. The ring serves to reduce the diameter of the annulus by allowing the leaflets to oppose each other normally and prevent redilation of the annulus. Sutures are used to attach a prosthetic ring to the annulus and to assist in plicating the annulus. This procedure, like heart valve replacement, is time-consuming. If the ring is severely malpositioned, then the stitches must be removed and the ring repositioned relative to the valve annulus during restitching. In other cases, a less than optimum annuloplasty may be tolerated by the surgeon rather than lengthening the time of surgery to restitch the ring.

WO0215798 describes an apparatus and method for stapling a prosthesis in-situ that includes an adjustable annuloplasty band and a positioning instrument for holding and positioning the band in a heart valve annulus. The instrument includes a curvature adjustable portion at one end for adjusting the band to conform to the heart valve annulus. Another end of the instrument, opposite the one end, includes a curvature adjustment actuating means. A stapling device attaches the band to the annulus simultaneously with the band being held in the annulus by the positioning instrument.

W00228321 describes an annuloplasty repair segment and template for heart valve annulus repair. The elongate flexible template may form a distal part of a holder that also has a proximal handle. Alternatively, the template may be releasably attached to a mandrel that slides within a delivery sheath, the template being released from the end of the sheath to enable manipulation by a surgeon. A tether connecting the template and mandrel may also be provided. The template may be elastic, temperature responsive, or multiple linked segments. The template may be aligned with the handle and form a two- or three-dimensional curve out of alignment with the handle such that the annuloplasty repair segment attached thereto conforms to the curve. The template may be actively or passively converted between its straight and curved positions.

### Summary of the Invention

In one aspect of the present invention, an apparatus for remodelling the annulus of a cardiac valve comprises a first elongate flexible member having a distal end portion and a proximal end portion interconnected by a main body portion. The distal end portion is steerable from the proximal end portion and is advanceable to one side of the annulus of the cardiac valve via a percutaneous approach. The distal end portion includes a terminal end and a connection section spaced apart by a predetermined length. The connecting section has means for disconnecting from the main body portion. The connecting section additionally includes means for interconnecting with the terminal end to form a ring of a predetermined shape and diameter adjacent the one side of the annulus to the cardiac valve. The distal end portion further comprises means for attaching to the annulus of the cardiac valve so that, when the terminal end and the connecting section are interconnected to form the ring, the distal end portion contracts and thereby remodels the annulus of the cardiac valve.

A percutaneous method for remodelling the annulus of a cardiac valve is described as background information that is useful for understanding the invention. An apparatus comprising a first elongate flexible member having a distal end portion and a proximal end portion interconnected by a main body portion is provided. The distal end portion is steerable from the proximal end portion and includes a terminal end and a connecting section spaced apart by a predetermined length. The connecting section has means for disconnecting from the main body portion. The connecting section further includes means for interconnecting with the terminal end to form a ring of a predetermined shape and diameter, and the distal end portion additionally includes means for attaching to the annulus to the cardiac valve. The apparatus is inserted into a patient's vasculature and the distal end portion is advanced to one side of the annulus of the cardiac valve. The distal end portion is then attached to one side of the annulus with the means for attaching. The terminal end and the connecting section.are interconnected adjacent the one side of the annulus of the cardiac valve to form the ring, whereby the distal end portion contracts in length and remodels the annulus of the cardiac valve. Thereafter, the connecting section is disconnected from the main body portion and the main body portion is retracted from the patient's vasculature.

## Brief Description of the Drawings

The foregoing and other features of the present invention will become apparent to those skilled in the art to which the present invention relates upon reading the following description with reference to the accompanying drawings, in which:

Fig. 1 is a cross-sectional view of an apparatus for remodeling the annulus of a cardiac valve constructed in accordance with the present invention;
Fig. 2 is a cross-sectional schematic view of a human heart;
Fig. 3 is a magnified perspective view showing a terminal end of the apparatus shown in Fig. 1 interconnected to a connecting section, and a main body portion disconnected from the connecting section;
Fig. 4 is a magnified perspective view of the apparatus of Fig. 1 showing the terminal end disconnected from the connecting section;
Fig. 5 is an alternate embodiment of the apparatus shown in Fig. 1;
Fig. 6A is a perspective view showing the distal end portion of the apparatus of Fig. 1 adjacent the annulus of the cardiac valve;
Fig. 6B is a cross-sectional view of the apparatus of Fig. 6A showing an attachment means;
Fig. 7A is a perspective view showing the distal end portion of the apparatus of Fig. 1 attached to the annulus of the cardiac valve;
Fig. 7B is a cross-sectional view of the apparatus showing the attachment means in a deployed configuration;
Fig. 8 is a perspective view showing the apparatus of Fig. 1 extending through an aorta to an inferior aspect of a mitral valve annulus;
Fig. 9 is a perspective view showing the apparatus of Fig. 1 positioned adjacent the inferior aspect of the mitral valve annulus;
Fig. 10 is a perspective view showing the apparatus of Fig. 1 attached to the mitral valve annulus;
Fig. 11 is a perspective view showing an alternate embodiment of an apparatus for remodeling the annulus of a cardiac valve positioned adjacent the mitral valve annulus; and
Fig. 12 is a perspective view showing the apparatus of Fig. 11 attached to the annulus of the mitral valve.

## Detailed Description

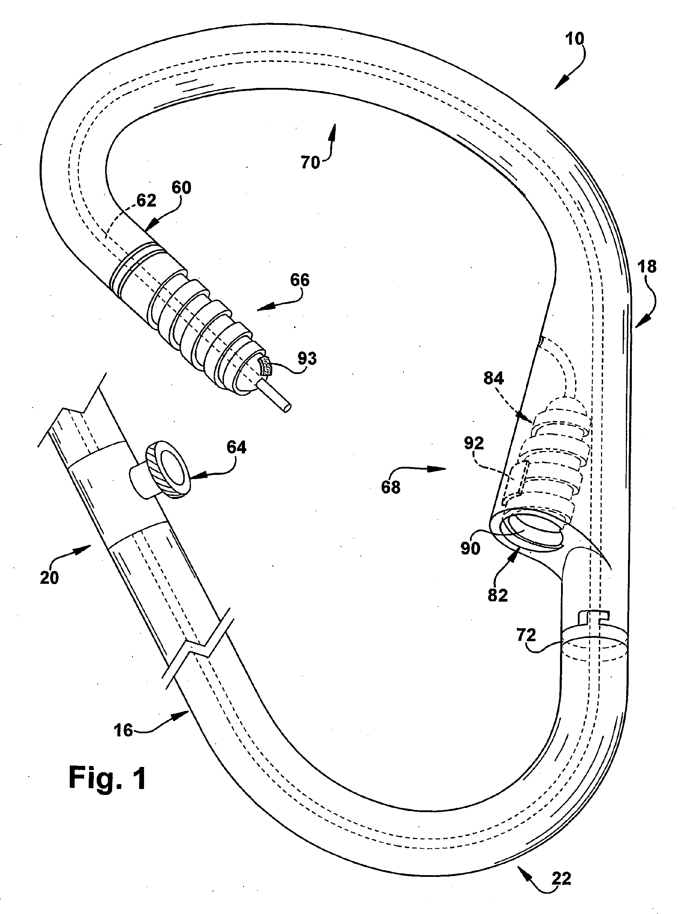

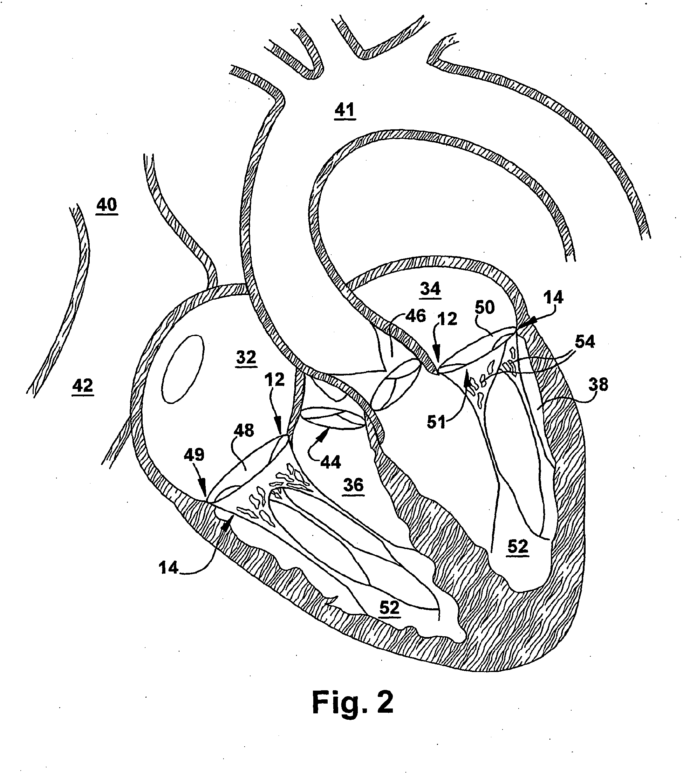

The present invention relates generally to cardiovascular valve repair, and more particularly to an apparatus and method for remodeling a cardiac valve annulus. As representative of the present invention, Fig. 1 illustrates an apparatus 10 for remodeling the annulus 12 (Fig. 2) of a cardiac valve 14. The apparatus 10 (Fig. 1) comprises a first elongate flexible member 16 having a distal end portion 18 and a proximal end portion 20 interconnected by a main body portion 22 capable of forming a ring which, when contracted, is capable of remodeling the annulus 12 of the cardiac valve 14.

Fig. 2 shows a human heart 30. The human heart 30 contains four chambers: the right and left atria 32 and 34 and the right and left ventricles 36 and 38. The thin-walled right atrium 32 receives deoxygenated blood from the superior vena cava 40, the inferior vena cava 42, and from the coronary sinus (not shown). The thin-walled left atrium 34 receives oxygenated blood from pulmonary veins (not shown). The left and right ventricles 38 and 36 pump oxygenated and deoxygenated blood, respectively, throughout the body, and the pocket-like pulmonary and aortic semilunar valves 44 and 46 prevent reflux into the ventricles. Atrial blood is pumped through the atrioventricular orifices, guarded by the 3-cusp tricuspid valve 48 on the right and the 2-cusp mitral valve 50 on the left. The mitral and tricuspid valves 48 and 50 are secured to the papillary muscles 52 in the left and right ventricles 38 and 36 by tendinous chordae tendineae 54 and by the mitral and tricuspid valve annuluses 51 and 49, areas of heart wall tissue at the junction of the atrial and ventricular walls that are relatively fibrous and significantly stronger than leaflet tissue.

One embodiment of the present invention is illustrated in Fig. 1. The first elongate flexible member 16 comprises a catheter ring (or sheath) 60 and a wire catheter (or guide) 62 disposed within the catheter ring. The catheter ring 60 may be flexible or rigid. For example, the catheter ring 60 may be comprised of multiple segments (not shown) configured such that the catheter ring is deformable by tensioning a tensioning member (not shown) coupled to the segments. Alternatively, the catheter ring 60 may be formed from an elastic material having a geometry selected to engage and optionally shape or constrict the annulus 12 of the cardiac valve 14. For instance, the catheter ring 60 may be formed from super-elastic material, shape memory alloy such as Nitinol, spring stainless steel, or the like. In other instances, the catheter ring 60 can be made of a rigid material such as hardened plastic, silicon, polyurethane, or the like. Alternatively, the catheter ring 60 may be selectively rigidified *in situ*, such as with a gooseneck or lockable element shaft (not shown), or any other rigidifying structure.

The wire catheter 62 may be disposed within the first elongate flexible member 16 and may be advanced or retracted throughout the first elongate flexible member. The wire catheter 62 may be comprised of a flexible, resiliently yieldable material such as stainless steel alloys, plastic polymers, carbon fiber, or the like. The position of the wire catheter 62 may be controlled from the proximal end portion 20 of the first elongate flexible member 16 using a rotational control mechanism 64. For instance, the rotational control mechanism 64 may be used to tension the wire catheter 62 so that the first elongate flexible member 16 is steerable through a patient's vasculature.

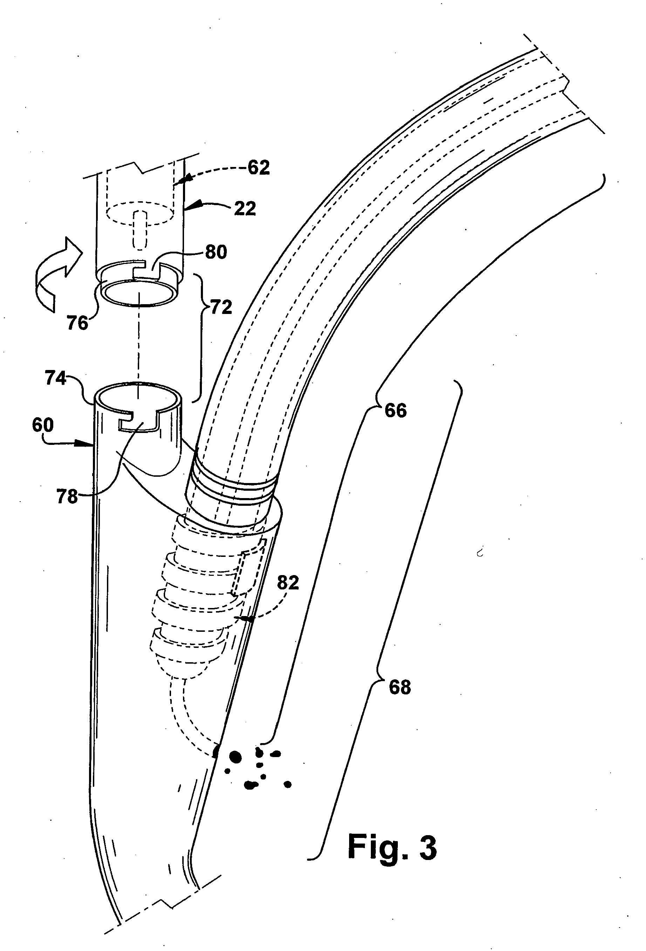

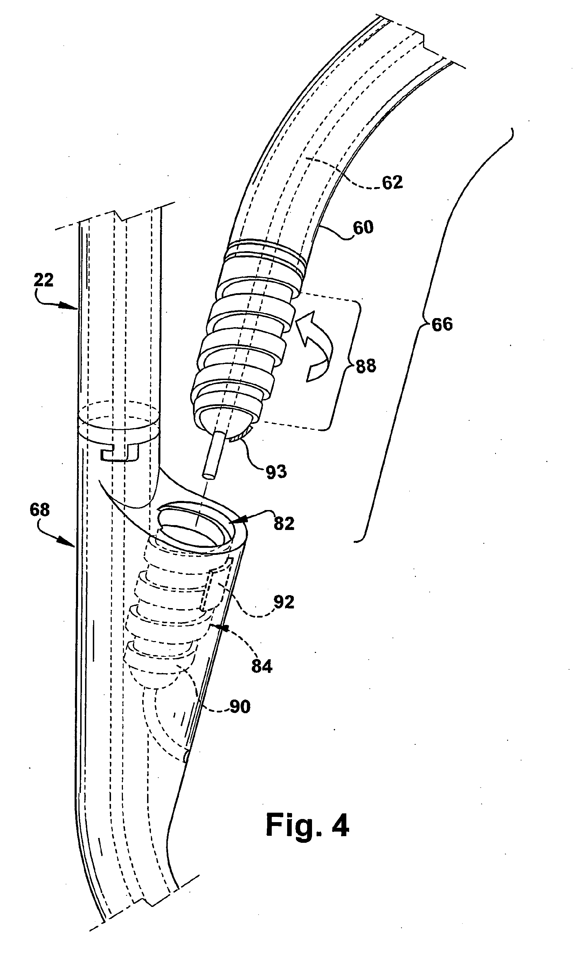

The distal end portion 18 of the first elongate flexible member 16 includes a terminal end 66 a connecting section 68 spaced apart by a predetermined length 70. Referring to Fig. 3, the connecting section 68 includes means 72 for disconnecting from the main body portion 22. For instance, the means 72 for disconnecting from the main body portion 22 can comprise first and second ring members 74 and 76 respectively attached to the connecting section 68 and the main body portion. The first ring member 74 can have a female portion 78 capable of mating with a male portion 80 of the second ring member 76 (Fig. 4). When separation of the connecting section 68 from the main body portion 22 is desired, the main body portion, the connecting section, or both can be rotated or unlocked so that the male portion 80 of the second ring member 76 is displaced from and thus no longer mated to the female portion 78 of the first ring member 74.

Alternative means 72 for disconnecting from the main body portion 22 are also possible. For instance, the means 72 for disconnecting can comprise at least two magnetic members (not shown) each securely attached to the connecting section 68 and the main body portion 22. When appropriately positioned, the magnetic members may be magnetically attracted so that the connecting section 68 and the main body portion 22 are joined. Then, when an appropriate amount of force (*e.g.*, torque) is applied to the main body portion 22, the connecting section 68, or both, the magnetic members may be sufficiently distanced so that the connecting section and the main body portion are separated.

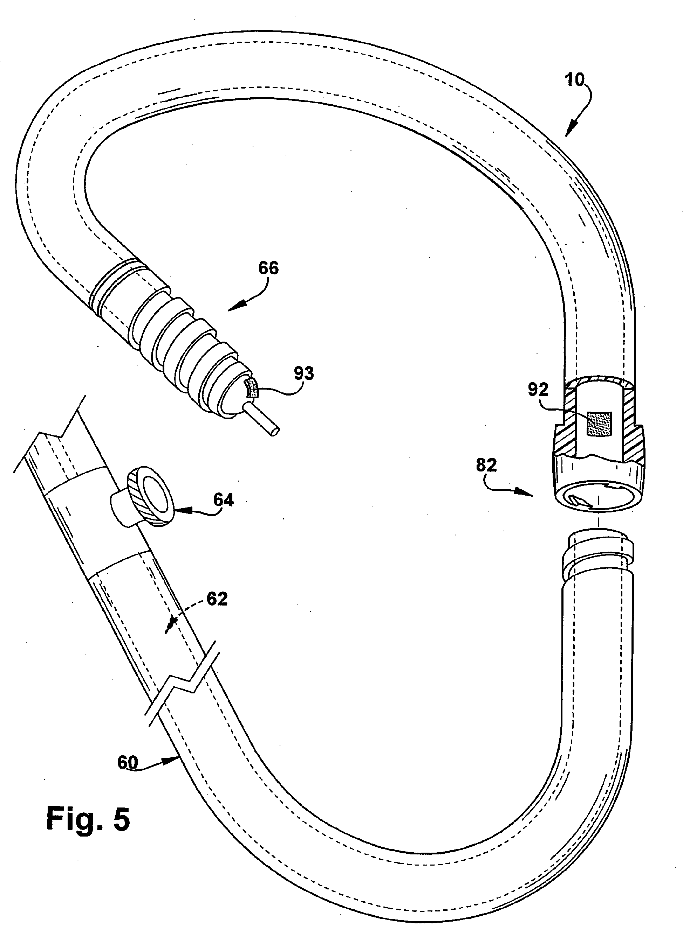

The connecting section 68 further includes means 82 for interconnecting with the terminal end 66. As shown in Fig. 4, the means 82 for interconnecting with the terminal end 66 can include a receptacle portion 84. Alternatively, when the wire catheter 62 is removed from the catheter ring 60 and the main body portion 22 is disconnected from the connecting section 68, the means 82 for interconnecting can comprise the end of the connecting section as shown in Fig. 5. The receptacle portion 84 in Fig. 4 has a shape complementary to the shape of the terminal end 66 such that the receptacle portion is capable of mating with the terminal end. Additionally, the receptacle portion 84 can include at least one first magnet 92.

As is also shown in Fig. 4, the terminal end 66 may have a shape complementary to the shape of the receptacle portion 84 such that the terminal end is capable of mating with the receptacle portion. For instance, the terminal end 66 may include a threaded portion 88 capable of mating with the receptacle portion 84 (which may also have a threaded portion 90 complementary in shape to the terminal end). Additionally, the terminal end 66 may include at least one second magnet 93.

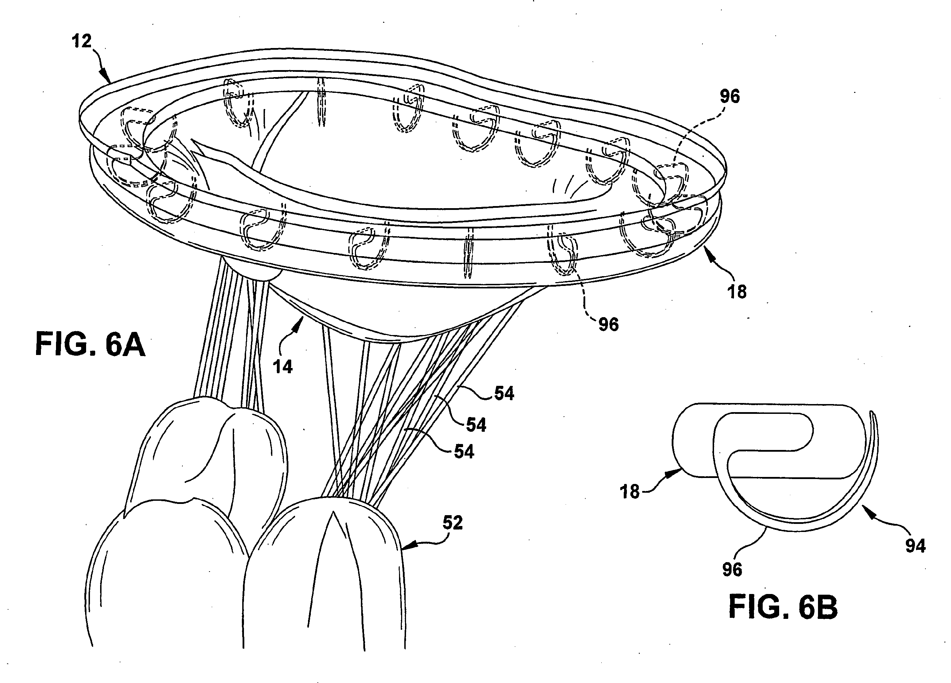

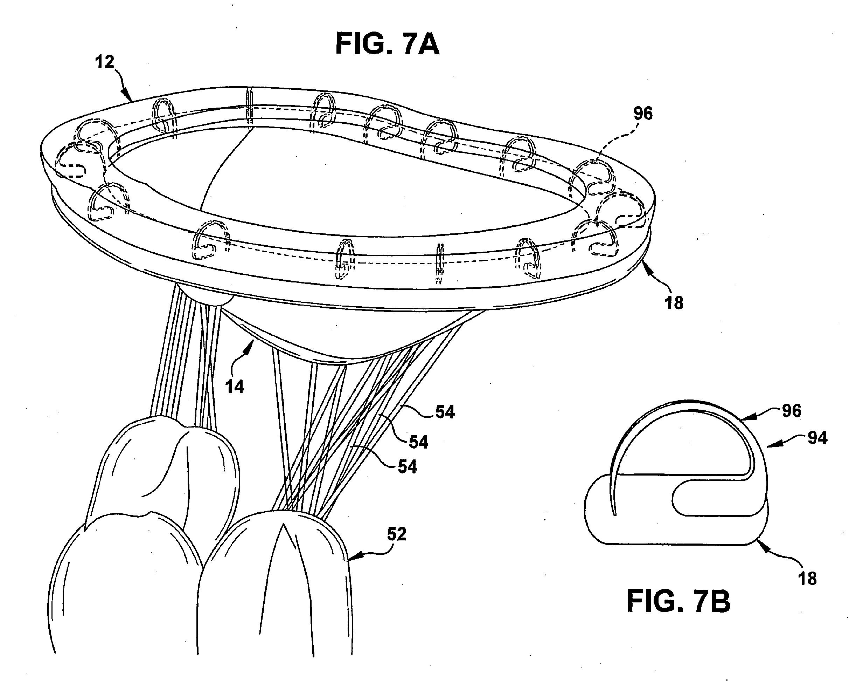

Referring to Figs. 6A, 6B, 7A and 7B, the distal end portion 18 of the first elongate flexible member 16 includes means 94 for attaching to the annulus 12 of the cardiac valve 14. The means 94 for attaching comprises a plurality of deployable hook members 96. The deployable hook members 96 can include any type of fastener, including, for example, C-shaped or semicircular hooks, curved hooks of other shapes, straight hooks, barbed hooks, clips of any kind, T-tags, or any other suitable fastener. The deployable hook members 96 may be made of any suitable material, such as super-elastic or shape-memory material like Nitinol or spring stainless steel. Additionally, the deployable hook members 96 may be made of a magnetic or electromagnetic material such as iron, NdFeB, SmCo and Alnico.

The deployable hook members 96 are integrally secured to the distal end portion 18. By "deployable" it is meant that the deployable hook members 96 can change from a first non-deployed shape (Fig. 6B) to a second deployed shape (Fig. 7B). When deployed, the deployable hook members 96 may change shape, enter the annulus 12 of the cardiac valve 14, and attach to the annulus of the cardiac valve. Thus, a crimping device or other similar mechanism is not required on the distal end portion 18 to apply force to the deployable hook members 96 and attach the deployable hook members to the annulus 12 of the cardiac valve 14.

Although the first elongate flexible member 16 is shown having a circular cross-sectional shape in Fig. 1, the first elongate flexible member may have any other suitable shape. For example, it may be advantageous to provide a first elongate flexible member 16 having an ovoid or elliptical cross-sectional shape (not shown). Such a shape may help ensure that the first elongate flexible member 16 is aligned, when positioned in a corner formed by a ventricular wall and a valve leaflet, so that the distal end portion 18 is optimally oriented to deliver the deployable hook members 96 into the annulus 12 of the cardiac valve 14. To further enhance contacting to the annulus 12 of the cardiac valve 14 and/or orientation of the first elongate flexible member 16, an expandable member (not shown) may also be coupled to the first elongate flexible member which expands to urge or press the first elongate flexible member into the corner formed by the ventricle wall and the valve leaflet.

The present invention also provides a percutaneous method for remodeling the annulus 12 of a cardiac valve 14. In one embodiment of the method, a first step comprises accessing the annulus 12 of the cardiac valve 14 and measuring the dimensions of the annulus of the cardiac valve. Access to the annulus 12 of the cardiac valve 14 may be accomplished by any available technique, including intravascular (described below), transthoracic, and the like. The method of the present invention will typically entail gaining access to a beating heart; however, the present invention may also be used for intravascular stopped-heart access as well as stopped-heart open chest procedures.

Prior to use of the apparatus 10, the physician will need to determine the dimensions of the annulus 12 of the cardiac valve 14. Various methods and devices for determining the dimensions of the annulus 12 of the cardiac valve 14 are known in the art, such as echocardiogram, computed tomography (CT), magnetic resonance imaging (MRI), fluoroscopy, and angiography. After determining the dimensions of the annulus 12 of the cardiac valve 14, the physician will select an appropriately-sized first elongate flexible member 16 by choosing a first elongate flexible member having a suitably sized pre-determined length 70. The pre-determined length 70 is suitably sized so that the dimensions of the distal end portion 18 of the first elongate flexible member 16 correspond to the dimensions of the annulus 12 of the cardiac valve 14. For instance, the physician may select a first elongate flexible member 16 having a pre-determined length 70 that is suitably sized so as to form a distal end portion 18 having a circumference approximately equal to the circumference of the annulus 12 of the cardiac valve 14.

Next, the physician inserts the first elongate flexible member 16 into the patient's vasculature. Where an intravascular approach is desired, the physician may insert the first elongate flexible member 16 through a femoral artery or vein (not shown). Alternatively, the physician may insert the first elongate flexible member 16 through the internal jugular vein (not shown). Additionally, the physician may choose to insert the first elongate flexible member 16 using a transthoracic approach by introducing the first elongate flexible member into the heart 30 via a minimally invasive incision or port on the heart wall.

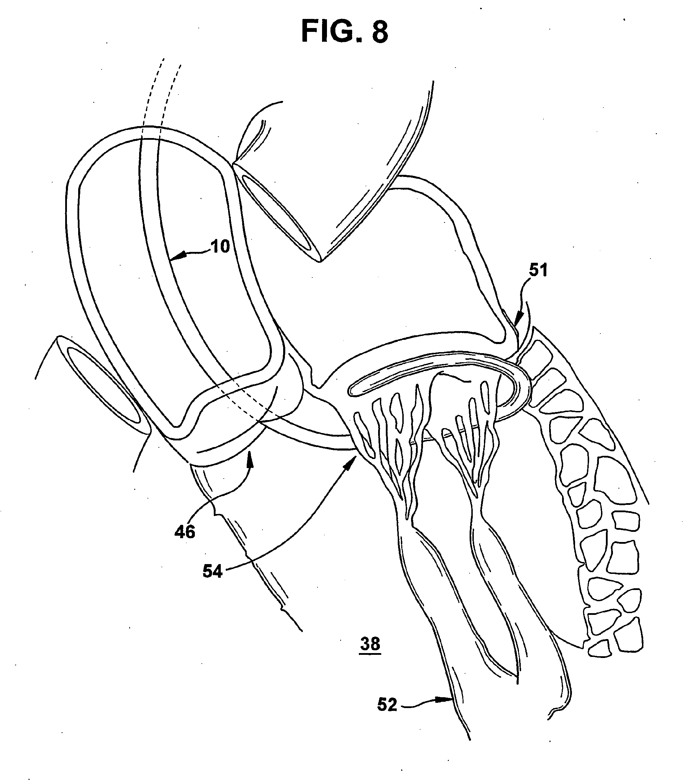

Once the physician has inserted the first elongate flexible member 16 into the patient's vasculature, the first elongate flexible member is urged toward one side of the annulus 12 of the cardiac valve 14. More particularly, the first elongate flexible member 16 may be urged toward a subvalvular space in the left ventricle 38, roughly defined as the space bordered by the inner surface of the ventricular wall, the inferior surface of the valve leaflets, and chordae tendineae 54 connected to the ventricular wall and leaflets. Where the physician desires to place the first elongate flexible member 16 at the inferior aspect of the annulus 51 of the mitral valve 50 via an intravascular approach, the physician may urge the first elongate flexible member in a retrograde fashion through a femoral artery (not shown), across the aortic arch 41, through the aortic valve 46, and then under one or more mitral valve leaflets in a subvalvular position within the left ventricle 38 (Fig. 8).

Alternatively, the physician may access the internal jugular vein (not shown) and then place the first elongate flexible member 16 at the superior aspect of the annulus 51 of the mitral valve 50 by urging the first elongate flexible member through the superior vena cava 40 into the right atrium 32, across the interatrial septum (not shown) to the left atrium 34, and then around the mitral annulus. The physician may also gain access to the superior aspect of the annulus 51 of the mitral valve 50 via a femoral vein (not shown) by urging the first elongate flexible member 16 through the inferior vena cava 42 into the right atrium 32, through the interatrial septum, into the left atrium 34, and around the mitral annulus.

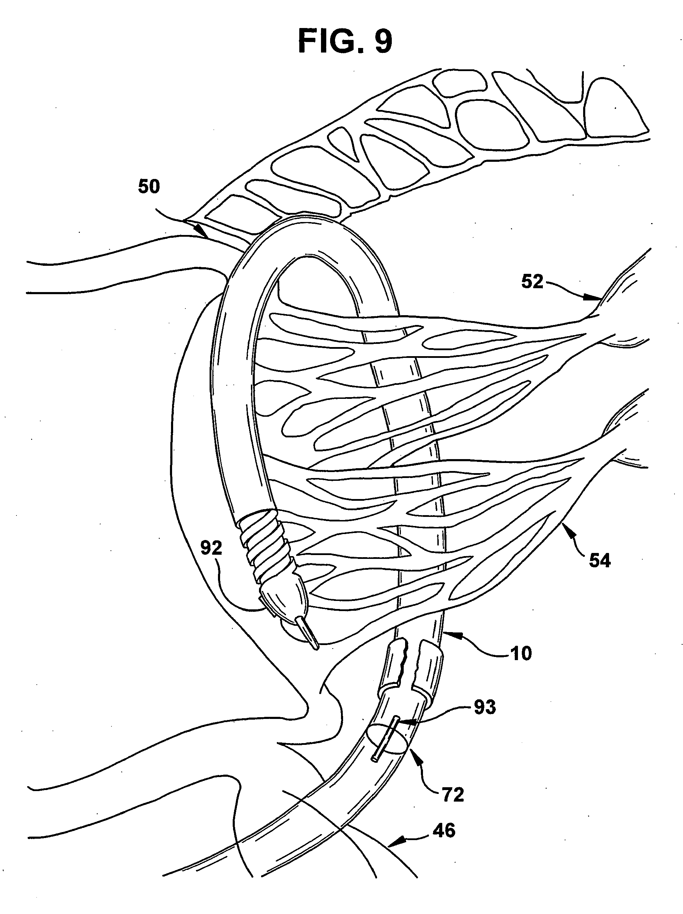

Once the first elongate flexible member 16 has been suitably positioned in the left ventricle 38, the terminal end 66 is guided toward the connecting section 68. When the terminal end 66 is positioned adjacent the connecting section 68 (Fig. 9), the second magnet 93 of the terminal end is magnetically attracted to the first magnet 92 of receptacle portion 84 so that the terminal end and the connecting section are magnetically held in contact. Consequently, the distal end portion 18 of the first elongate flexible member 16 is formed into a circular or annular shape. The physician then positions the distal end portion 18 adjacent the inferior aspect of the annulus 51 of the mitral valve 50.

Next, the physician attaches the distal end portion 18 to the annulus 51 of the mitral valve 50 by deploying the deployable hook members 96. The deployable hook members 96 may be deployed in a variety of ways. For instance, the deployable hook members 96 may be deployed via an electromagnetic mechanism (not shown). The electromagnetic mechanism can comprise an electromagnetic wire (not shown) operably linked to the deployable hook members 96. The electromagnetic wire can comprise the wire catheter 62 or, alternatively, an additional wire. The electromagnetic wire may also be operably linked to a battery or other power source capable of generating electricity. When electricity is supplied to the electromagnetic wire, the deployable hook members 96 change from a non-deployed configuration to a deployed configuration.

The deployable hook members 96 may also be deployed via a camming mechanism (not shown). The camming mechanism may comprise a tensioning wire (not shown) operably linked to the deployable hook members 96. When the tensioning wire is manipulated by the physician, the deployable hook members 96 change from a non-deployed configuration to a deployed configuration. Alternatively, the deployable hook members 96 may be deployed as a result of the first elongate flexible member 16 assuming a particular confirmation (*e.g.*, by changing the first elongate flexible member from a linear configuration to a circular or annular configuration). Also, an inflatable member (not shown) disposed within the first elongate flexible member 16 may deploy the deployable hook members 96 when inflated.

After the distal end portion 18 of the first elongate flexible member 16 is attached to the annulus 51 of the mitral valve 50, the terminal end 66 is securely interconnected with the connecting section 68 to form a ring. The terminal end 66 is securely interconnected to the connecting section 68 by manipulating the terminal end so that the threaded portion 88 is progressively threaded into the threaded portion 90 of the receptacle portion 84. As the terminal end 66 is progressively threaded into the receptacle portion 84, the diameter of the distal end portion 18 progressively shrinks so that the annulus 51 of the mitral valve 50 is remodeled. It is contemplated that the physician may use image guidance (*e.g.*, MRI, CT, fluoroscopy, ultrasound, angiogram, or combinations thereof) to monitor the degree to which the diameter of the distal end portion 18 is shrinking as the terminal end 66 is threaded into the receptacle portion 84.

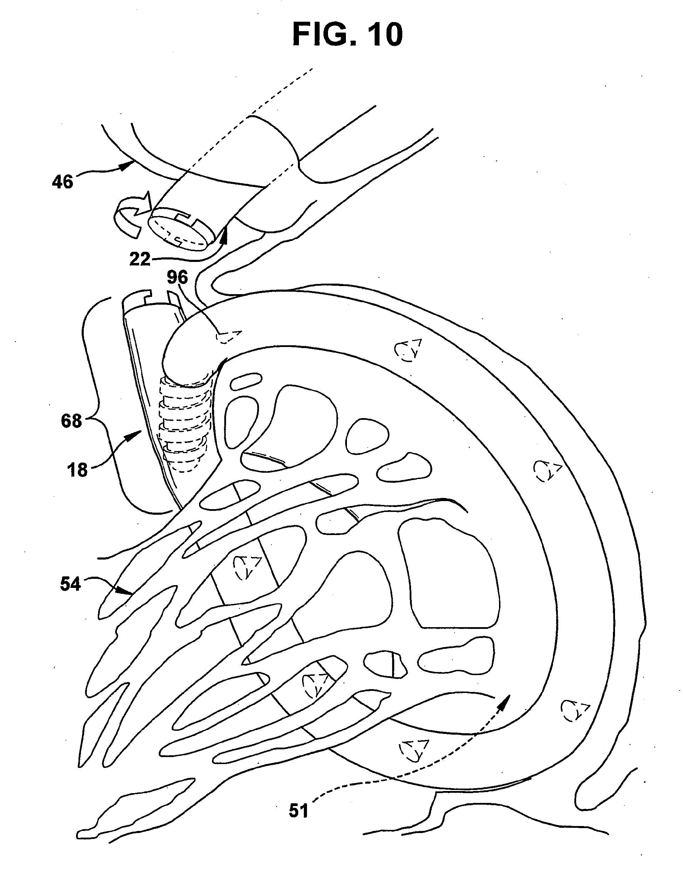

After the terminal end 66 and the connecting section 68 of the first elongate flexible member 16 are securely interconnected, the physician may disconnect the connecting section from the main body portion 22. As shown in Fig. 10, the main body portion 22 may be disconnected from the connecting section 68 by applying a force (*e.g.*, torque) sufficient to separate the first and second ring members 74 and 76. After the connecting section 68 and the main body portion 22 are disconnected, the physician may retract the main body portion and complete the medical procedure. With the distal end portion 18 of the first elongate flexible member 16 now securely attached to the annulus 51 of the mitral valve 50, the annulus of the mitral valve is successfully remodeled and the patient is provided with a normally functioning mitral valve.

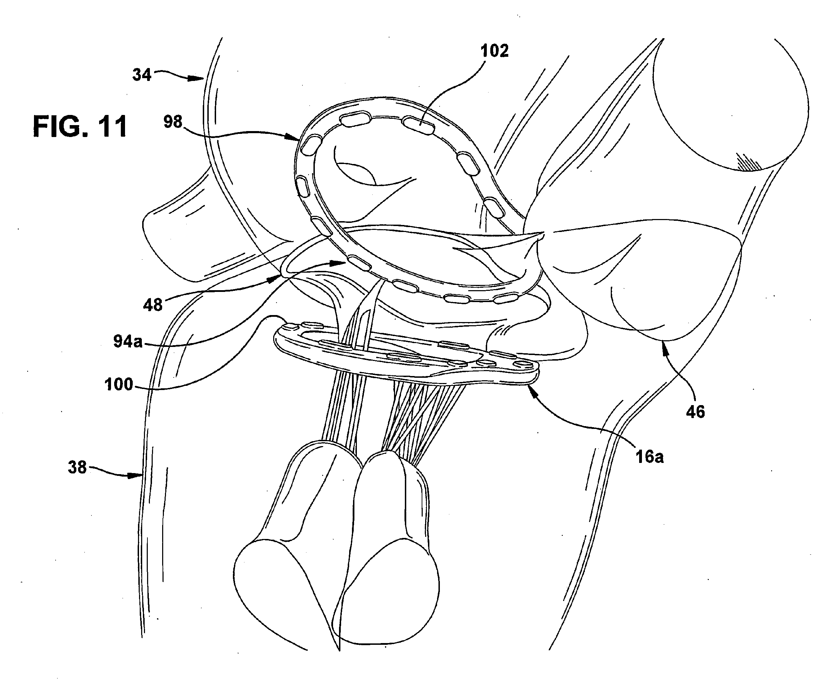

Illustrated in Fig. 11 is another embodiment of the present invention comprising a first elongate flexible member $16_a$ and a second elongate flexible member 98. The first elongate flexible member $16_a$ and the second elongate flexible member 98 are identically constructed as the apparatus 10 shown in Fig. 1, except where as described below. In Fig. 8, structures that are identical as structures in Fig. 1 use the same reference numbers, whereas structures that are similar but not identical carry the suffix "a". Also in Fig. 8, the connecting section 68 of the first and second elongate flexible members $16_a$ and 98 has been omitted for clarity.

The distal end portion 18 of the first and second elongate flexible members $16_a$ and 98 comprises means $94_a$ for attaching to the annulus 12 of a cardiac valve 14. As shown in Fig. 11, the means $94_a$ for attaching of the first and second elongate flexible members $16_a$ and 98 respectively comprise at least one magnetic element 100 and 102. The at least one magnetic element 100 of the first elongate flexible member $16_a$ can have any size and shape, and be securely attached to the distal end portion 18 of the first elongate flexible member. Additionally, the at least one magnetic element 102 of the second elongate flexible member 98 can have any size and shape, and be securely attached to the distal end portion 18 of the second elongate flexible member. The means $94_a$ for attaching may be comprised of a material capable of producing a magnetic field. Examples of suitable materials include iron, NdFeB, SmCo and Alnico.

Although the first and second elongate flexible members $16_a$ and 98 are shown having a circular cross-sectional shape in Fig. 1, the first and second elongate flexible members may have any other suitable shape. For example, it may be advantageous to provide first and second elongate flexible members $16_a$ and 98 having an ovoid or elliptical cross-sectional shape (not shown). Such a shape may help ensure that the distal end portion 18 of the first and second elongate flexible members $16_a$ and 98 is aligned, when positioned in a corner formed by a ventricular wall and a valve leaflet, so that the distal end portion is optimally oriented to attach to the annulus 12 of the cardiac valve 14. To further enhance contacting of the annulus 12 of the cardiac valve 14 and/or orientation of the first and second elongate flexible members $16_a$ and 98, an expandable member (not shown) may also be coupled to the first and second elongate flexible members. For example, the expandable member may urge or press the first elongate flexible member 16 into the corner formed by the left ventricular wall and a mitral valve leaflet. Alternatively, the expandable member may urge or press the second elongate flexible member 98 into the corner formed by the left atrial wall and the mitral valve annulus 51.

It should be appreciated by the skilled artisan that at least a portion of the present invention may be made from a bioabsorbable material including, for example, magnesium alloy, dendrimers, biopolymers such as thermoplastic starch, polyalctides, cellulose, and aliphatic aromatic copolyesters.

Additionally, it should be appreciated that at least a portion of the present invention may be covered by a layer (not shown) of biocompatible material. The layer of biocompatible material may be synthetic such as Dacron® (Invista, Wichita, KS), woven velour, polyurethane, polytetrafluoroethylene (PTFE), expanded PTFE, Gore-Tex® (W. L. Gore & Associates, Flagstaff, AZ), or heparin-coated fabric. Alternatively, the layer may be a biological material such as bovine or equine pericardium, peritoneal tissue, an allograft, a homograft, a patient graft, or a cell-seeded tissue. The layer can cover, for example, the entire surface of the first elongate flexible member 16 or, alternatively, only the distal end portion 18. The layer may be attached around the entire circumference of the first elongate flexible member 16, for example, or, alternatively, may be attached in pieces or interrupted sections. By covering at least a portion of the present invention with a layer of biocompatible material, the hemocompatability of the present invention is improved.

At least a portion of the present invention may be treated with at least one therapeutic agent for eluting into cardiac tissue or a cardiac chamber. The therapeutic agent is capable of preventing a variety of pathological conditions including, but not limited to, arrhythmias, thrombosis, stenosis and inflammation. Accordingly, the therapeutic agent may include at least one of an anti-arrhythmic agent, anticoagulant, an antioxidant, a fibrinolytic, a steroid, an anti-apoptotic agent, and/or an anti-inflammatory agent. Optionally or additionally, the therapeutic agent may be capable of treating or preventing other disease or disease processes such as microbial infections and heart failure. In these instances, the therapeutic agent may include an inotropic agent, a chronotropic agent, an anti-microbial agent, and/or a biological agent such as a cell or protein. More specific types of these therapeutic agents are listed below, including other types of therapeutic agents not discussed above.

A plurality of portions of the present invention may be separately treated with a different one of the therapeutic agents. In the embodiment of the present invention shown in Fig. 11, for example, the first and second elongate flexible members $16_a$ and 98 may be separately treated with an anti-inflammatory agent and an anti-coagulant, respectively. Alternatively, only the distal end portion 18 of the first elongate flexible member 16 may be treated with a chronotropic agent. By treating the present invention with different therapeutic agents, different medical conditions can be simultaneously treated. It should be appreciated that the present invention may be treated with any combination and/or variation of the therapeutic agents mentioned above and discussed further below.

Examples of acceptable therapeutic agents include heparin, coumadin, synthetic heparin analogues (*e.g.*, fondaparinux), G(GP) $II_b$/$III_a$ inhibitors, vitronectin receptor antagonists, hirudin, antithrombin III, drotrecogin alpha; fibrinolytics such as alteplase, plasmin, lysokinase, factor XIIa, factor VIIa, prourokinase, urokinase, streptokinase; thrombocyte aggregation inhibitors such as ticlopidine, clopidogrel, abciximab, dextrans; corticosteroids such as aldlometasones, estradiols, such as 17β-estradiol, amcinonides, augmented betamethasones, beclomethasones, betamethasones, budesonides, cortisones, clobetasol, clocortolones, desonides, desoximetasones, dexamethasones, flucinolones, fluocinonides, flurandrenolides, flunisolides, fluticasones, halcinonides, halobetasol, hydrocortisones, methylprednisolones, mometasones, prednicarbates, prednisones, prednisolones, triamcinolones; fibrinolytic agents such as tissue plasminogen activator, streptokinase, dipyridamole, ticlopidine, clopidine, and abciximab; non-steroidal anti-inflammatory drugs such as salicyclic acid and salicyclic acid derivatives, para-aminophenol derivatives, indole and indene acetic acids (*e.g.*, etodolac, indomethacin, and-sulindac), heteroaryl acetic acids (*e.g.*, ketorolac, diclofenac, and tolmetin), arylpropionic acids (*e.g.*, ibuprofen and derivatives thereof), anthranilic acids (*e.g.*, meclofenamates and mefenamic acid), enolic acids (*e.g.*, piroxicam, tenoxicam, phenylbutazone, and oxyphenthatrazone), gold compounds (*e.g.*, auranofin, aurothioglucose, and gold sodium thiomalate), diflunisal, meloxicam, nabumetones, naproxen, oxaprozin, salsalate, celecoxib, rofecoxib; cytostatics such as alkaloids and podophyllum toxins such as vinblastin, vincristin; alkylants such as nitrosoureas and nitrogen lost analogues; cytotoxic antibiotics such as daunorubicin, doxorubicin, and other anthracyclins and related substances, bleomycin, and mitomycin; antimetabolites such as folic acid analogues, purine analogues and related inhibitors (*e.g.*, mercaptopurine, thioguanine, pentostatin, and 2-chlorodeoxyadenosine), pyrimidine analogues (*e.g.*, fluorouracil, floxuridine, and cytarabine), and platinum coordination complexes (*e.g.*, cisplatinum, carboplatinum and oxaliplatinum); tacrolimus, azathioprine, cyclosporine, paclitaxel, docetaxel, sirolimus; amsacrin, irinotecan, imatinib, topotecan, interferon-alpha 2a, interferon-alpha 2b, hydroxycarbamide, miltefosin, pentostatin, porfimer, aldesleukin, bexarotene, and tretinoin; antiandrogens and antiestrogens; antiarrythmics, in particular antiarrhythmics of class I such as antiarrhythmics of the quinidine type (*e.g.*, quinidine, dysopyramide, ajmaline, prajmalium bitartrate, and detajmium bitartrate); antiarrhythmics of the lidocaine type, (*e.g.*, lidocaine, mexiletin, phenyloin, and tocainid); antiarrhythmics of class I C (*e.g.*, propafenone, flecainide (acetate)); antiarrhythmics of class II, including betareceptor blockers such as metoprolol, esmolol, propranolol, metoprolol, atenolol, and oxprenolol; antiarrhythmics of class III such as amiodarone and sotalol; anti arrhythmics of class IV such as diltiazem, and verapamil; and other antiarrhythmics such as adenosine, orciprenaline, and ipratropium bromide.

Other types of therapeutic agents may include digitalis glycosides such as acetyl digoxin/methyldigoxin, digitoxin, and digoxin; heart glycosides such as ouabain and proscillaridin; antihypertensives such as centrally effective antiadrenergic substances (*e.g.*, methyldopa and imidazoline receptor agonists); calcium channel blockers of the dihydropyridine type, such as nifedipine and nitrendipine; ACE inhibitors (*e.g.*, quinaprilate, cilazapril, moexipril, trandolapril, spirapril, imidapril, and trandolapril); angiotensin-II-antagonists (*e.g.*, candesartancilexetil, valsartan, telmisartan, olmesartan medoxomil, and eprosartan); peripherally effective alpha-receptor blockers such as prazosin, urapidil, doxazosin, bunazosin, terazosin, and indoramin; vasodilators such as dihydralazine, diisopropyl amine dichloroacetate, minoxidil, and nitropiusside-sodium; other antihypertonics such as indapamide, codergocrin mesilate, dihydroergotoxin methane sulphonate, cicletanin, bosentan, and fluorocortisone; phosphodiesterase inhibitors, such as milrinone and enoximone, as well as antihypotonics (*e.g.*, adrenergics and dopaminergic substances such as dobutamine, epinephrine, etilefrine, norfenefrine, norepinephrine, oxilofrine, dopamine, midodrine, pholedrine, and amezinium methyl) and partial adrenoreceptor agonists (*e.g.*, dihydroergotamine); fibronectin, polylysines and ethylene vinyl acetates; and adhesive substances such as cyanoacrylates, beryllium, and silica.

Additional therapeutic agents may also include antibiotics and antiinfectives such as -lactam antibiotics (*e.g.*, -lactamase-sensitive penicillins, including benzyl penicillins (penicillin G) and phenoxymethylpenicillin (penicillin V)); -lactamase-resistant penicillins, such as aminopenicillins, which include amoxicillin, ampicillin, and bacampicillin; acylaminopenicillins such as mezlocillin and piperacillin; carboxypenicillines and cephalosporins (*e.g.*, cefazolin, cefuroxim, cefoxitin, cefotiam, cefaclor, cefadroxil, cefalexin, loracarbef, cefixim, cefuroximaxetil, ceftibuten, cefpodoximproxetil, and cefpodoximproxetil); aztreonam, ertapenem, and meropenem; -lactamase inhibitors such as sulbactam and sulfamicillintosilates; tetracyclines such as doxycycline, minocycline, tetracycline, chlorotetracycline, oxytetracycline;

aminoglycosides such as gentamicin, neomycin, streptomycin, tobramycin, amikasin, netilmicin, paromomycin, framycetin, and spectinomycin; makrolide antibiotics such as azithromycin, clarithromycin, erythromycin, roxithromycin, spiramycin, and josamycin; lincosamides such as clindamycin and lincomycin; gyrase inhibitors, such as fluoroquinolones, which include ciprofloxacin, ofloxacin, moxifloxacin, norfloxacin, gatifloxacin, enoxacin, fleroxacin, and levofloxacin; quinolones such as pipemidic acid; sulphonamides such as trimethoprim, sulphadiazin, and sulphalene; glycopeptide antibiotics such as vancomycin and teicoplanin; polypeptide antibiotics, such as polymyxins, which include colistin, polymyxin-b, and nitroimidazol derivatives (*e.g.,* metronidazol and tinidazol); aminoquinolones such as chloroquin, mefloquin, and hydroxychloroquin; biguanides such as proguanil; quinine alkaloids and diaminopyrimidines such as pyrimethamine; amphenicols such as chloramphenicol; rifabutin, dapsone, fusidinic acid, fosfomycin, nifuratel, telithromycin, fusafungin, fosfomycin, pentamidindiisethionate, rifampicin, taurolidine, atovaquone, and linezolid; virostatics such as aciclovir, ganciclovir, famciclovir, foscamet, inosine (dimepranol-4-acetamidobenzoate), valganciclovir, valaciclovir, cidofovir, and brivudin; tyrosine kinase inhibitors; anti-apoptotic agents such as caspase inhibitors (*e.g.,* fluoromethylketone peptide derivatives), calpain inhibitors, cathepsin inhibitors, nitric oxide synthase inhibitors, flavonoids, vitamin A, vitamin C, vitamin E, vitamin D, pycnogenol, super oxidedismutase, N-acetyl cysteine, selenium, catechins, alpha lipoic acid, melatonin, glutathione, zinc chelators, calcium chelators, and L-arginine; Coumadin; beta-blockers; diuretics; spirolactone; TC-313; and natural products such as vinca alkaloids (*e.g.*, vinblastine, vincristine and vinorelbine).

As noted above, the therapeutic agent may also include a biological agent. The biological agent may include organic substances such as peptides, proteins, enzyme, carbohydrates (*e.g.*, monosaccharides, oligosaccharides and polysacchardies), lipids, phospholipids, steroids, lipoproteins, glycoproteins, glycolipids, proteoglycans, polynucleotides (*e.g.*, DNA and RNA), antisense polynucleotides (*e.g.*, c-myc antisense), antibodies (*e.g.*, monoclonal or polycolonal) and/or antibody fragments (*e.g.*, anti-CD34 antibody), bioabsorbable polymers (*e.g.*, polylactonic acid), chitosan, extracellular matrix modulators, such as matrix metalloproteinases (MMP), which include MMP-2, MMP-9 and Batimastat; and protease inhibitors.

Biological agents may include, for example, agents capable of stimulating angiogenesis in the myocardium. Such agents may include vascular endothelial growth factor (VEGF), basic fibroblast growth factor (bFGF), non-viral DNA, viral DNA, and endothelial growth factors (*e.g.*, FGF-1, FGF-2, VEGF, TGF). Other growth factors may include erythropoietin and/or various hormones such as corticotropins, gonadotropins, sonlatropin, thyrotrophin, desmopressin, terlipressin, oxytocin, cetrorelix, corticorelin, leuprorelin, triptorelin, gonadorelin, ganirelix, buserelin, nafarelin, and goserelin. Additional growth factors may also include cytokines, epidermal growth factors (EGF), platelet derived growth factor (PDGF), transforming growth factors- (TGF-), transforming growth factor- (TGF-), insulin-like growth factor-I (IGF-I), insulin-like growth factor-II (IGF-II), interleukin-1 (IL-1), interleukin-2 (IL-2), interleukin-6 (IL-6), interleukin-8 (IL-8), tumour necrosis factor- (TNF-), tumour necrosis factor-(TNF-), interferon- (INF-), colony stimulating factors (CSFs); monocyte chemotactic protein, and fibroblast stimulating factor 1.

Still other biological agents may include regulatory peptides such as somatostatin and octreotide; bisphosphonates (*e.g.*, risedronates, pamidronates, ibandronates, zoledronic acid, clodronic acid, etidronic acid, alendronic acid, and tiludronic acid); fluorides such as disodium fluorophosphate and sodium fluoride; calcitonin and dihydrotachystyrene; histamine; fibrin or fibrinogen; endothelin-1; angiotensin II; collagens; bromocriptin; methylsergide; methotrexate; carbontetrachloride and thioacetamide.

The present invention may also be treated (*i.e.*, seeded) with other biological agents, such as cells. Suitable cells may include any one or combination of eukaryotic cells. Additionally or optionally, the cells may be capable of producing therapeutic agents and/or genetically engineered to produce therapeutic agents. Suitable cells for use in the present invention include, for example, progenitor cells such as adult stem cells, embryonic stem cells, and umbilical cord blood stem cells. The cells may be autologous or allogenic, genetically engineered or non-engineered, and may include, for example, mesenchymal or mesodermal cells, including, but not limited to, endothelial progenitor cells, endothelial cells, and fibroblasts. Mixtures of such cells can also be used.

A variety of *ex vivo* or *in vivo* methods can be used to deliver a nucleic acid molecule or molecules, such as a gene or genes, to the cells. For example, the cells can be modified (*i.e.*, genetically engineered) to produce or secrete any one or combination of the above therapeutic agents, including, but not limited to, anticoagulant agents, antiplatelet agents, antifibrinolytic agents, angiogenesis factors, and the like. *Ex vivo* gene transfer is a process by which cells are removed from the body using well known techniques, genetically manipulated, usually through transduction or transfection of a nucleic acid molecule into the cells *in vitro*, and the returned to the body for therapeutic purposes. This contrasts with *in vivo* genetic engineering where a gene transfer vector is administered to a patient resulting in genetic transfer into cells and tissues in the intact patient. *Ex vivo* and *in vivo* gene transfer techniques are well known to one of skill in the art.

To treat the present invention with at least one therapeutic agent, a variety of methods, agents, and compositions may be used. For example, the therapeutic agent can be simply linked to the stent surface, embedded and released from within polymer materials, such as a polymer matrix, or surrounded by and released through a carrier. Several approaches to treating medical devices with therapeutic agents exist. Some therapeutic agents can be loaded directly onto metallic surfaces; however, a coating composition, typically comprised of at least one polymer and at least one therapeutic agent, is usually used to treat drug-eluting devices. The coating composition ensures retention of the therapeutic agent during deployment and modulates elution kinetics of the therapeutic agent. By altering the release kinetics of different therapeutic agents in the same coating composition, distinct phases of a given disease process may be targeted.

The present invention may be treated with a coating composition comprising at least one therapeutic agent and at least one dendrimer, polymer or oligomer material. The dendrimer(s), polymer(s) and/or oligomer(s) may be of various types and from various sources, including natural or synthetic polymers, which are biocompatible, bioabsorbable, and useful for controlled release of the therapeutic agent. For example, synthetic polymers can include polyesters, such as polylactic acid, polyglycolic acid, and/or combinations thereof, polyanhydrides, polycaprolactones, polyhydroxybutyrate valerates, and other biodegradable polymers or mixtures of copolymers thereof. Natural polymeric materials can include proteins such as collagen, fibrin, elastin, extracellular matrix components, other biologic agents, and/or mixtures thereof.

The polymer material or mixture thereof of the coating composition can be applied with the therapeutic agent on the surface of the present invention and can comprise a single layer. Optionally, multiple layers of the polymer material can be applied to form the coating composition. Multiple layers of the polymer material can also be applied between layers of the therapeutic agent. For example, the polymeric layers may be applied sequentially, with the first layer directly in contact with the uncoated surface of the apparatus and a second layer comprising the therapeutic agent and having one surface in contact with the first layer and the opposite surface in contact with a third layer of polymeric material which is in contact with the surrounding tissue. Additional layers of the polymeric material and therapeutic agent can be added as required.

Alternatively, the coating composition can be applied as multiple layers comprising one or more therapeutic agents surrounded by polymer material. For instance, the coating composition can comprise multiple layers of a.single therapeutic agent, one or more therapeutic agents in each layer, and/or differing therapeutic agents in alternating layers. Alternatively, the layers comprising the therapeutic agent can be separated from one another by a layer of polymer material.

The coating composition may further comprise at least one pharmaceutically acceptable polymers and/or pharmaceutically acceptable carriers, for example, non-absorbable polymers, such as ethylene vinyl acetate and methylmethacrylate. The non-absorbable polymer, for example, can aid in further controlling release of the therapeutic agent by increasing the molecular weight of the coating composition and thereby delaying or slowing the rate of release of the therapeutic agent.

The coating composition can be applied to the present invention using standard techniques to cover the entire surface of the apparatus, or partially, as a single layer in a dot matrix pattern, for example. The coating composition can be applied using various techniques available in the art, such as dipping, spraying, vapor deposition, an injection-like and/or a dot matrix-like approach. Upon contact of the coating composition with adjacent tissue where implanted, the coating composition can begin to degrade in a controlled manner. As the coating composition degrades, the therapeutic agent is slowly released into adjacent tissue and the therapeutic agent is eluted so that the therapeutic agent can have its effect locally.

Where the therapeutic agent comprises a biological agent, such as cells, the biological agent can be coated directly onto the surface of the present invention or, alternatively, they can be incorporated into the polymeric material (*e.g.*, into a polymer matrix). Such biological agents may also be included within at least one microscopic containment vehicle (*e.g.*, a liposome, nanocapsule, nanoparticle, micelle, synthetic phospholipid, gas-dispersion, emulsion, microemulsion, nanosphere, and the like) that can be stimulated to release the biological agent(s) and/or that release the biological agent(s) in a controlled manner. The microscopic containment vehicle can be coated onto the surface of the present invention or incorporated into the polymeric material. Where the biological agent comprises cells, for example, the cells can be induced to produce, activate, and/or release their cellular products (including one or more therapeutic agents) by an external stimulation device (*e.g.*, an electrical impulse). Alternatively, cells can constitutively release one or more therapeutic agents at a desired level.

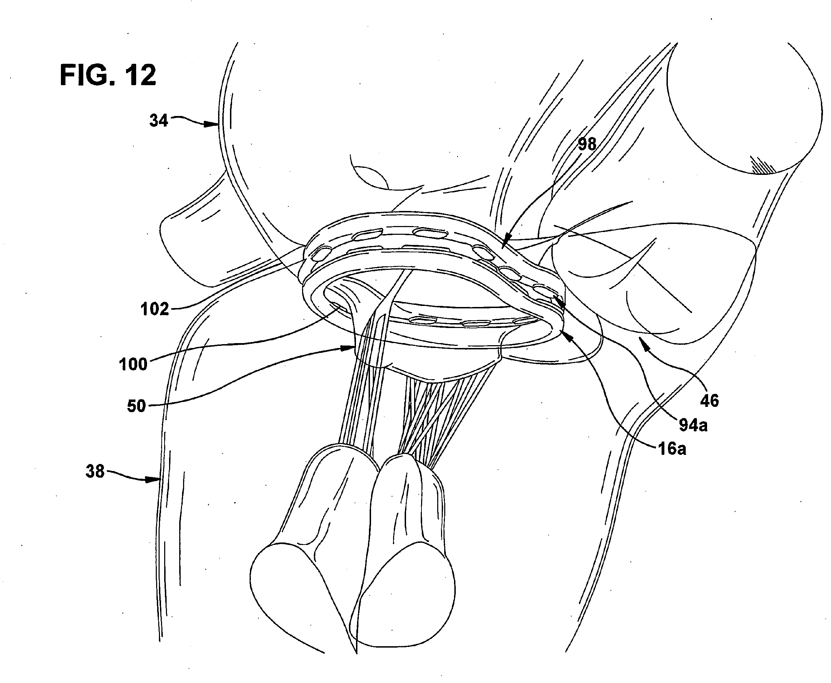

Referring to Figs. 11 and 12, an alternate embodiment of the method of the present invention comprises using first and second elongate flexible members $16_a$ and 98 to remodel the annulus 51 of a mitral valve 50. A first step of the method comprises accessing the annulus 51 of the mitral valve 50 and measuring the dimensions of the annulus of the mitral valve. Access to the annulus 51 of the mitral valve 50 may be accomplished by any available technique, including intravascular (discussed below), transthoracic, minimally invasive or surgical approach, and the like. The method of the present invention will typically entail gaining access to a beating heart; however, the present invention may also be used for intravascular stopped-heart access as well as stopped-heart open chest procedures.

After measuring the dimensions of the annulus 51 of the mitral valve 50, the physician selects appropriately-sized first and second elongate flexible members $16_a$ and 98. More particularly, the physician selects first and second elongate flexible members $16_a$ and 98 each having a suitably sized pre-determined length 70. The pre-determined length 70 may be suitably sized so that the dimensions of the distal end portion 18 of each of the first and second elongate flexible members $16_a$ and 98 respectively correspond to the dimensions of the inferior and superior aspects of the annulus 51 of the mitral valve 50. For instance, the physician may select a first elongate flexible member $16_a$ having a pre-determined length 70 that is suitably sized so as to form a distal end portion 18 having a circumference approximately equal to the circumference of the inferior aspect of the annulus 51 of the mitral valve 50.

The physician next inserts the first elongate flexible member $16_a$ into a patient's vasculature as described above. After inserting the first elongate flexible member $16_a$, the physician then inserts the second elongate flexible member 98 into the patient's vasculature. For instance, the physician may insert the second elongate flexible member 98 into a femoral vein (not shown) and then urge the second elongate flexible member through the inferior vena cava 42, into the right atrium 32, across the interatrial septum (not shown), and then into the left atrium 34.

After delivering the first and second elongate flexible members $16_a$ and 98 to the inferior and superior aspects of the annulus 51 of the mitral valve 50 (respectively), the terminal end 66 of each of the first and second elongate flexible members is guided toward the connecting section 68 of each of the first and second elongate flexible members. When the terminal end 66 of each of the first and second elongate flexible members $16_a$ and 98 is positioned adjacent the connecting section 68 of each of the first and second elongate flexible members, the second magnet 93 of the terminal end is magnetically attracted to the first magnet 92 of the receptacle portion 84 so that the distal end of each of the first and second elongate flexible members is magnetically held in contact with the connecting section of each of the first and second elongate flexible members. Consequently, the distal end portion 18 of each of the first and second elongate flexible members $16_a$ and 98 is formed into a circular or annular shape.

Next, the physician respectively positions the distal end portion 18 of each of the first and second elongate members $16_a$ and 98 adjacent the inferior and superior aspects of the annulus 51 of the mitral valve 50. The at least one magnetic element 100 of the first elongate flexible member $16_a$ is then magnetically attracted to the at least magnetic element 102 of the second elongate flexible member 98 so that the distal end portion 18 of each of the first and second elongate flexible members are pulled together. Consequently, the annulus 51 of the mitral valve 50 is tightly fit between the first and second elongate flexible members $16_a$ and 98.

The following computation may be used to determine the appropriate amount of magnetic force between the first and second elongate members $16_a$ and 98. The first and second elongate flexible members $16_a$ and 98 may be represented by the following diagram:

The Maxwell's stress tensor can be written as follows:

$$T_{ij} = \frac{1}{\mu}[B_i B_j - \frac{1}{2}B^2 \delta_{ij}]$$

Since only $\vec{B}_z$ exit, the Maxwell's stress tensor can be written as:

$$T_{ij} = \begin{bmatrix} -\dfrac{|B_z|^2}{2\mu} & 0 & 0 \\[2ex] 0 & -\dfrac{|B_z|^2}{2\mu} & 0 \\[2ex] 0 & 0 & \dfrac{|B_z|^2}{2\mu} \end{bmatrix}$$

The stress tensor vector which is normal to the surface in two-dimensional coordinates has the form:

$$P = \begin{bmatrix} -\dfrac{|B_z|^2}{2\mu} & 0 & 0 \\[2ex] 0 & -\dfrac{|B_z|^2}{2\mu} & 0 \\[2ex] 0 & 0 & \dfrac{|B_z|^2}{2\mu} \end{bmatrix} \begin{pmatrix} 0 \\ 0 \\ n_z \end{pmatrix} = \dfrac{|B_z|^2}{2\mu}$$

Since the distance between the first and second elongate flexible members $16_a$ and 98 is small (3-5 mm), the loss of magnetic flux density may be neglected when $|B_z| = 0.5$ T. The pressure can be calculated as follows:

$$P = \frac{|B_z|^2}{2\mu} = \frac{0.5^2}{8\pi \times 10^{-7}} = 99.47 \ (\text{KPa})$$

When $area = 2 \times 10^{-3} \times 2 \times \pi \times 30 \times 10^{-3} = 3.77 \times 10^{-4}$ m$^2$, the magnetic force may be calculated as follows:

$$F = P \times area = 99.47 \times 10^3 \times 3.77 \times 10^{-4} = 37.5 \ (\text{Newton})$$

This foregoing computation provides an estimate of the force necessary to anchor the first and second elongate flexible members $16_a$ and 98 to the annulus 12. Other similar computations can be readily performed where the present invention has similar geometries and/or dimensions.

The terminal end 66 of each of the first and second elongate flexible members $16_a$ and 98 is then respectively interconnected with the connecting section 68 of each of the first and second elongate flexible members to form separate rings. The terminal end 66 of each of the first and second elongate flexible members $16_a$ and 98 is securely interconnected to the connecting section 68 of each of the first and second elongate flexible members by manipulating the terminal end so that the threaded portion 88 of each of the first and second elongate flexible members is progressively threaded into the threaded portion 90 of each of the first and second elongate flexible members. As the terminal end 66 of each of the first and second elongate flexible members $16_a$ and 98 is progressively threaded into the receptacle portion 84 of each of the first and second elongate flexible members, the diameter of the distal end portion 18 of each of the first and second elongate flexible members progressively shrinks so that the annulus 51 of the mitral valve 50 is remodeled. It is contemplated that the physician may use image guidance (*e.g.*, MRI, CT, fluoroscopy, ultrasound, angiogram, or combinations thereof) to monitor the degree to which the distal end portion 18 of each of the first and second elongate flexible members $16_a$ and 98 is shrinking as the terminal end 66 of each of the first and second elongate flexible members is threaded into the receptacle portion 84 of each of the first and second elongate flexible members.

After the terminal end 66 and the connecting section 68 of each of the first and second elongate flexible members $16_a$ and 98 are securely interconnected, the physician may disconnect the connecting section of each of the first and second elongate flexible members from the main body portion 22 of each of the first and second elongate flexible members. After the connecting section 68 and the main body portion 22 of each of the first and second elongate

flexible members 16$_a$ and 98 are disconnected, the physician may retract the main body portion 22 of each of the first and second elongate flexible members and complete the procedure. With the distal end portion 18 of each of the first and second elongate flexible members 16$_a$ and 98 now securely attached to the inferior and superior aspects of the annulus 51 of the mitral valve 50 (respectively), the mitral valve annulus is successfully remodeled.

From the above description of the invention, those skilled in the art will perceive improvements, changes and modifications. The skilled artisan should appreciate that the methods of the present invention are not limited to the order of steps described herein. For instance, the second elongate flexible member 98 may be placed at the superior aspect of the annulus 12 of the cardiac valve 14 before placing the first elongate flexible member 16$_a$ at the inferior aspect of the annulus of the cardiac valve. Additionally, the skilled artisan should appreciate that the apparatus 10 may be used to remodel the annulus 49 of a tricuspid valve 48. Additionally, the skilled artisan should appreciate that other approaches may be used to place the present invention. For example, the present invention may be placed via a minimally invasive, transthoracic approach via a port on the heart wall or, alternatively, via an open-chest procedure under direct vision. Such improvements, changes and modifications within the skill of the art are intended to be covered by the appended claims.

**Claims**

1.   An apparatus (10) for remodelling the annulus (12) of a cardiac valve (14), said apparatus (10) comprising:

a first elongate flexible member (16) having a distal end portion (18) and a proximal end portion (20) interconnected by a main body portion (22), said distal end portion (18) being steerable from said proximal end portion (20), said distal end portion (18) being advanceable to one side of the annulus (12) of the cardiac valve (14) via a percutaneaous approach;
said distal end portion (18) including a terminal end (66) and a connecting section (68) spaced apart by a predetermined length, said connection section (68) having means (72) for disconnecting from said main body portion (22), said connecting section (68) further having means (82) for interconnecting with said terminal end (66) to form a ring of a predetermined shape and diameter adjacent the one side of the annulus (12) of the cardiac valve (14);
said distal end portion (18) further including means (94) for attaching to the annulus (12) of the cardiac valve (14) so that, when said terminal end (66) and said connecting section (68) are interconnected to form said ring the distal end portion (18) contracts and thereby remodels the annulus (12) of the cardiac valve (14).

2.   The apparatus (10) of claim 1 wherein said means (94) for attaching to the annulus (12) of the cardiac valve (14) comprises a plurality of deployable hook members (96).

3.   The apparatus (10) of claim 1 wherein said terminal end (66) of said distal end portion (18) further comprises a threaded portion (88).

4.   The apparatus (10) of claim 1 wherein said connecting section (68) of said distal end portion (18) further comprises a receptacle portion (84) having a shape complementary to the shape of the said threaded portion (88).

5.   The apparatus (10) of claim 1 further comprising:

a second elongate flexible member (98) having a distal end portion (18) and a proximal end portion (20) interconnected by a main body portion (22), said distal end portion (18) being steerable from said proximal end portion (20), said distal end portion (18) being advanceable to an opposite side of the annulus (12) of she cardiac valve (14) via a percutaneous approach;
said distal end portion (18) including a terminal end (66) and a connecting section (68) spaced apart by a predetermined length, said connecting section (68) having means (72) for disconnecting from said main body portion (22), said connecting section (68) further having means (82) for interconnecting with said terminal end (66) to form a ring adjacent the opposite side of the annulus (12) of the cardiac valve (14);
said distal end portion (18) further including means (94a) for attaching to the annulus (12) of the cardiac valve (14) so that, when said terminal end (66) and said connecting section (68) are interconnected, the distal end portion (18) contracts and thereby remodels the annulus (12) of the cardiac valve (14);
said means (94a) for attaching to the annulus (12) of the cardiac valve (14) comprises at least one magnetic element (100) on said first elongate flexible member (16a) and at least one magnetic element (102) on said second elongate flexible member (98), said magnetic elements (100, 102) being magnetically attracted to one another so that, when said apparatus (10) is place in the annulus (12) of the cardiac valve (14), said first (16a) and second (98) elongate flexible members are pulled toward one another to attach to the annulus (12);

6. The apparatus (10) of claim 5 wherein at least a portion of said first (16a) and second (98) elongate flexible members is covered with a layer of biocompatible material.

7. The apparatus (10) of claim 5 wherein at least a portion of the said first (16a) and second (98) elongate flexible members is covered with a layer of bioabsorbable material.

8. The apparatus (10) of claim 5 wherein at least a portion of said first (16a) and second (98) elongate flexible members is treated with at least one therapeutic agent for eluting into cardiac tissue or a cardiac chamber.

9. The apparatus (10) of claim 5 wherein a plurality of portions of said first (16a) and second (98) elongate flexible members are separately treated with a different one of said at least one therapeutic agent.

10. The apparatus (10) of claim 9 wherein said first elongate flexible member (16a) and said second elongate flexible member (98) are each separately treated with a different one of said at least one therapeutic agent.

**Patentansprüche**

1. Vorrichtung (10) zur Ummodellierung des Rings (12) einer Herzklappe (14), wobei die Vorrichtung (10) aufweist:

ein erstes langgestrecktes flexibles Element (16) mit einem distalen Endabschnitt (18) und einem proximalen Endabschnitt (20), die durch einen Hauptkörperabschnitt (22) miteinander verbunden sind, wobei der distale Endabschnitt (18) von dem proximalen Endabschnitt (20) aus steuerbar ist, wobei der distale Endabschnitt (18) über einen perkutanen Zugang zu einer Seite des Rings (12) der Herzklappe (14) vorgerückt werden kann; wobei der distale Endabschnitt (18) ein terminales Ende (66) und einen durch eine vorgegebene Länge beabstandeten Verbindungsabschnitt (68) aufweist, wobei der Verbindungsabschnitt (68) eine Einrichtung (72) zum Abtrennen von dem Hauptkörperabschnitt (22) aufweist, wobei der Verbindungsabschnitt (68) ferner eine Einrichtung (82) zum Verbinden mit dem terminalen Ende (66) aufweist, um angrenzend an die eine Seite des Rings (12) der Herzklappe (14) einen Ring von vorgegebener Form und vorgegebenem Durchmesser zu formen; wobei der distale Endabschnitt (18) ferner eine Einrichtung (94) zur Befestigung an dem Ring (12) der Herzklappe (14) aufweist, so daß, wenn das terminale Ende (66) und der Verbindungsabschnitt (68) miteinander verbunden sind, um den Ring zu formen, der distale Endabschnitt (18) sich zusammenzieht und **dadurch** den Ring (12) der Herzklappe (14) ummodelliert.

2. Vorrichtung (10) nach Anspruch 1, wobei die Einrichtung (94) zur Befestigung an dem Ring (12) der Herzklappe (14) mehrere einsetzbare Hakenelemente (96) aufweist.

3. Vorrichtung (10) nach Anspruch 1, wobei das terminale Ende (66) des distalen Endabschnitts (18) ferner einen Gewindeabschnitt (88) aufweist.

4. Vorrichtung (10) nach Anspruch 1, wobei der Verbindungsabschnitt (68) des distalen Endabschnitts (18) ferner einen Aufnahmeabschnitt (84) mit einer Form aufweist, die zu der Form des Gewindeabschnitts (88) komplementär ist.

5. Vorrichtung (10) nach Anspruch 1, die ferner aufweist:

ein zweites langgestrecktes flexibles Element (98) mit einem distalen Endabschnitt (18) und einem proximalen Endabschnitt (20), die durch einen Hauptkörperabschnitt (22) miteinander verbunden sind, wobei der distale Endabschnitt (18) von dem proximalen Endabschnitt (20) aus steuerbar ist, wobei der distale Endabschnitt (18) über einen perkutanen Zugang zu einer gegenüberliegenden Seite des Rings (12) der Herzklappe (14) vorgerückt werden kann; wobei der distale Endabschnitt (18) ein terminales Ende (66) und einen durch eine vorgegebene Länge beabstandeten Verbindungsabschnitt (68) aufweist, wobei der Verbindungsabschnitt (68) eine Einrichtung (72) zum Abtrennen von dem Hauptkörperabschnitt (22) aufweist, wobei der Verbindungsabschnitt (68) ferner eine Einrichtung (82) zum Verbinden mit dem terminalen Ende (66) aufweist, um angrenzend an die gegenüberliegende Seite des Rings (12) der Herzklappe (14) einen Ring zu formen;
wobei der distale Endabschnitt (18) ferner eine Einrichtung (94a) zur Befestigung an dem Ring (12) der Herzklappe (14) aufweist, so daß, wenn das terminale Ende (66) und der Verbindungsabschnitt (68) miteinander verbunden sind, der distale Endabschnitt (18) sich zusammenzieht und **dadurch** den Ring (12) der Herzklappe (14) ummodelliert;
wobei die Einrichtung (94a) zur Befestigung an dem Ring (12) der Herzklappe (14) mindestens ein magnetisches Element (100) an dem ersten langgestreckten flexiblen Element (16a) und mindestens ein magnetisches Element (102) an dem zweiten langgestreckten flexiblen Element (98) aufweist, wobei die magnetischen Elemente (100, 102) magnetisch zueinander angezogen werden, so daß, wenn die Vorrichtung (10) in den Ring (12) der Herzklappe (14) eingesetzt wird, das erste (16a) und zweite langgestreckte flexible Element (98) zueinander gezogen werden und an dem Ring (12) anhaften.

6. Vorrichtung (10) nach Anspruch 5, wobei zumindest ein Teil des ersten (16a) und zweiten langgestreckten flexiblen Elementes (98) mit einer Schicht aus biologisch verträglichem Material bedeckt ist.

7. Vorrichtung (10) nach Anspruch 5, wobei zumindest ein Teil des ersten (16a) und zweiten langgestreckten flexiblen Elementes (98) mit einer Schicht aus biologisch absorbierbarem Material bedeckt ist.

8. Vorrichtung (10) nach Anspruch 5, wobei zumindest ein Teil des ersten (16a) und zweiten langgestreckten flexiblen Elementes (98) mit mindestens einem therapeutischen Wirkstoff zur Elution in Herzgewebe oder eine Herzkammer behandelt wird.

9. Vorrichtung (10) nach Anspruch 5, wobei mehrere Abschnitte des ersten (16a) und zweiten langgestreckten flexiblen Elementes (98) getrennt mit einem anderen von dem mindestens einen therapeutischen Wirkstoff behandelt werden.

10. Vorrichtung (10) nach Anspruch 9, wobei das erste langgestreckte flexible Element (16a) und das zweite langgestreckte flexible Element (98) jeweils getrennt mit einem anderen von dem mindestens einen therapeutischen Wirkstoff behandelt werden.

## Revendications

1. Dispositif (10) de remodelage de l'anneau (12) d'une valve cardiaque (14), ledit dispositif (10) comprenant:

   un premier élément allongé flexible (16), comportant une partie d'extrémité distale (18) et une partie d'extrémité proximale (20) interconnectées par une partie de corps principal (22), ladite partie d'extrémité distale (18) pouvant être orientée à partir de ladite partie d'extrémité proximale (20), ladite partie d'extrémité distale (18) pouvant être avancée vers un côté de l'anneau (12) de la valve cardiaque (14) par une approche percutanée ; ladite partie d'extrémité distale (18) englobant une extrémité terminale (66) et une section de connexion (68) espacées d'une longueur prédéterminée, ladite section de connexion (68) comportant un moyen (72) pour assurer la déconnexion de ladite partie de corps principal (22), ladite section de connexion (68) comportant en outre un moyen (82) pour assurer l'interconnexion avec ladite extrémité terminale (66) pour former une bague d'une forme et d'un diamètre prédéterminés près dudit côté de l'anneau (12) de la valve cardiaque (14) ; ladite partie d'extrémité distale (18) englobant en outre un moyen (94) pour assurer la fixation sur l'anneau (12) de la valve cardiaque (14), de sorte que lorsque ladite extrémité terminale (66) et ladite section de connexion (68) sont interconnectées pour former ladite bague, la partie d'extrémité distale (18) est contractée, remodelant ainsi l'anneau (12) de la valve cardiaque (14).

2. Dispositif (10) selon la revendication 1, dans lequel ledit moyen (94) destiné à assurer la fixation sur l'anneau (12) de la valve cardiaque (14) comprend plusieurs éléments de crochet à déploiement (96).

3. Dispositif (10) selon la revendication 1, dans lequel ladite extrémité terminale (66) de ladite partie d'extrémité distale (18) comprend en outre une partie filetée (88).

4. Dispositif (10) selon la revendication 1, dans lequel ladite section de connexion (68) de ladite partie d'extrémité distale (18) comprend en outre une partie de réceptacle (84) ayant une forme complémentaire de la forme de ladite partie filetée (88).

**5.** Dispositif (10) selon la revendication 1, comprenant en outre :

un deuxième élément allongé flexible (98) comportant une partie d'extrémité distale (18) et une partie d'extrémité proximale (20) interconnectées par une partie de corps principal (22), ladite partie d'extrémité distale (18) pouvant être orientée à partir de ladite partie d'extrémité proximale (20), ladite partie d'extrémité distale (18) pouvant être avancée vers un côté opposé de l'anneau (12) de la valve cardiaque (14) par une approche percutanée ;

ladite partie d'extrémité distale (78) englobant une extrémité terminale (66) et une section de connexion (68) espacées d'une longueur prédéterminée, ladite section de connexion (68) comportant un moyen (72) destiné à assurer la déconnexion de ladite partie de corps principal (22), ladite section de connexion (68) comportant en outre un moyen (82) destiné à assurer l'interconnexion avec ladite extrémité terminale (66) pour former une bague adjacente au côté opposé de l'anneau (12) de la valve cardiaque (14) ;

ladite partie d'extrémité distale (18) englobant en outre un moyen (94a) destiné à assurer la fixation sur l'anneau (12) de la valve cardiaque (14), de sorte que lorsque ladite extrémité terminale (66) et ladite section de connexion (68) sont interconnectées, la partie d'extrémité distale (18) est contractée, remodelant ainsi l'anneau (12) de la valve cardiaque (14) ;

ledit moyen (94a) destiné à assurer la fixation à l'anneau (12) de la valve cardiaque (14) comprenant au moins un élément magnétique (100) sur ledit premier élément allongé flexible (16a) et au moins un élément magnétique (102) sur ledit deuxième élément allongé flexible (98), lesdits éléments magnétiques (100, 102) étant attirés magnétiquement l'un vers l'autre, de sorte que lorsque ledit dispositif (10) est placé dans l'anneau (12) de la valve cardiaque (14), lesdits premier (16a) et deuxième (98) éléments allongés flexibles sont tirés l'un vers l'autre en vue de la fixation sur l'anneau (12).

**6.** Dispositif (10) selon la revendication 5, dans lequel au moins une partie desdits premier (16a) et deuxième (98) éléments allongés flexibles est recouverte d'une couche d'un matériau biocompatible.

**7.** Dispositif (10) selon la revendication 5, dans lequel au moins une partie desdits premier (16a) et deuxième (98) éléments allongés flexibles est recouverte d'une couche d'un matériau bioabsorbable.

**8.** Dispositif (10) selon la revendication 5, dans lequel au moins une partie desdits premier (16a) et deuxième (98) éléments allongés flexibles est traitée avec au moins un agent thérapeutique en vue d'une élution dans les tissus cardiaques ou une cavité cardiaque.

**9.** Dispositif (10) selon la revendication 5, dans lequel plusieurs parties desdits premier (16a) et deuxième (98) éléments allongés flexibles sont traitées séparément avec un agent différent dudit au moins un agent thérapeutique.

**10.** Dispositif (10) selon la revendication 9, dans lequel ledit premier élément allongé flexible (16a) et ledit deuxième élément allongé flexible (98) sont chacun traités séparément avec un agent différent dudit au moins un agent thérapeutique.

**Fig. 1**

**Fig. 2**

**Fig. 3**

**Fig. 4**

**Fig. 5**

FIG. 6A

FIG. 6B

# FIG. 7A

# FIG. 7B

EP 1 903 991 B1

# FIG. 8

# FIG. 9

# FIG. 10

**FIG. 11**

34

102

98

48

94a

100

38

46

16a

EP 1 903 991 B1

FIG. 12

EP 1 903 991 B1

**EP 1 903 991 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0215798 A

- WO 0228321 A